# EUROPEAN PATENT APPLICATION

(11) **EP 2 889 051 A1**
(43) Date of publication of application: **01.07.2015**
(21) Application number: 13832642.6
(22) Date of filing: 07.08.2013
(51) Int. Cl.: A61M 5/34

(54) **INJECTION NEEDLE**

(30) Priority: 27.08.2012 JP 2012186949
(71) Applicant: Nanbu Plastics Co., Ltd., Shizuoka 421-0305 (JP)
(72) Inventor: INOU, Akinori, Haibara-gun Shizuoka 421-0305 (JP)
(74) Representative: UEXKÜLL & STOLBERG
(86) International application number: PCT/JP2013/004767
(87) International publication number: WO 2014/034032

(57) **Abstract**

An injection needle capable of supplying a drug solution to a tissue layer at a depth of interest, regardless of variation in the amount of adhesive applied for securing a needle, is provided.

An injection needle **A** is attached to a tip section of a syringe. The injection needle **A** has a hub **2** and needles **4** protruding out from the tip surface of the hub **2.** The hub **2** has a hub body **10,** a protruding section **11** formed at the tip section of the hub body **10,** and a flange. A plurality of longitudinal grooves **14** extending in the axial direction are formed in the perimeter of the protruding section **11.** A through hole **17** for allowing the needle **4** to pass through is formed in the hub body **10** so as to extend the longitudinal groove **14.** The needle **4** is fixed to the tip surface of the hub body **10** with a fixation agent **6,** with which inside of a recessed section **15** is filled. The tip of the needle **4** protrudes out forward more than a surface of the protruding section **11.**

## Description

### [Technical Field]

The present invention relates to an injection needle for percutaneous administration of a drug solution.

### [Background Art]

A syringe is conventionally used for the administration of a drug solution or a drug formulation (hereinafter, referred to as "drug solution") that cannot be orally administered. However, a method using a syringe has a problem in that the level of stress exerted to the body by such a method is significant and the stress is also accompanied by pain. Although percutaneous administration methods using an agent in a patch comprising a drug solution have also been used, such methods have issues in that the methods require a long time for drug efficacy to be expressed and types of usable drug solutions are limited.

In order to solve the above problems, Japanese Laid-Open Publication No. 2005-87521 (Patent Literature 1), Japanese National Phase PCT Laid-Open Publication No. 2010-508058 (Patent Literature 2) and Japanese Laid-Open Publication No. 2007-54194 (Patent Literature 3) propose percutaneous drug administering devices, which use a needle having a small diameter to alleviate pain due to puncture with a needle upon injection.

In these percutaneous drug administering devices, a needle is secured to the device by using an adhesive or the like so that the needle protrudes out from the surface of the tip of the device by a predetermined length. However, as shown in FIG. 1 of Patent Literature 3, since a solidified component of the adhesive remains on the surface of the tip of the device, the length of protrusion of the needle from the solidified component of the adhesive is affected by the size of the solidified component of the adhesive. For this reason, there is a shortcoming in that the length of protrusion of the needle would be inconsistent if the amount of adhesive applied varies.

In particular, for needles designed to have a short length in order to supply a drug solution at a shallow portion of an intradermal tissue, the size of a solidified component of an adhesive greatly affects whether the drug solution is supplied to a shallow intradermal tissue layer.

In order to overcome such a shortcoming, Japanese National Phase PCT Laid-open Publication No. 2009-511192 (Patent Literature 4) proposes a configuration of providing a recess to a portion to be applied with an adhesive so that the excessive amount of the adhesive is absorbed within the recess or the excessive amount of adhesive flows out externally from the recess.

However, an injection needle with such a configuration has a shortcoming in that a needle is readily bent when an excessive amount of adhesive is applied. Further, there is no description regarding providing a plurality of needles.

### [Citation List]

### [Patent Literature]

[PTL 1] Japanese Laid-Open Publication No. 2005-87521
[PTL 2] Japanese National Phase PCT Laid-open Publication No. 2010-508058
[PTL 3] Japanese Laid-Open Publication No. 2007-54194
[PTL 4] Japanese National Phase PCT Laid-open Publication No. 2009-511192

### [Summary of Invention]

### [Technical Problem]

The present invention is for solving the above-described problems. The objective of the present invention is to provide an injection needle capable of supplying a drug solution to an intradermal tissue layer at a depth of interest, regardless of the variation in the amount of adhesive applied for securing a needle.

Another objective of the present invention is to provide an injection needle capable of preventing deformation or the like of a needle by having a protruding section that protrudes out from a hub body to support the tip portion of a needle.

### [Solution to Problem]

The present invention has the following features for solving the above-described problem.

### [Item 1]

An injection needle to be attached to a tip section of a syringe including a hub and a plurality of needles protruding out from a tip surface of the hub, wherein:
the hub has a hub body formed in a generally tubular shape, a protruding section formed on a tip section of the hub body, and a flange formed at a base section of the hub body,
a plurality of longitudinal grooves extending in an axial direction are formed in the perimeter of the protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside of the recessed section is filled, and
a tip of the needle protrudes out forward more than a surface of the protruding section, and a base section of the needle faces a drug solution accommodating section formed inside the hub body.

### [Item 2]

The injection needle of item 1, wherein the longitudinal grooves are formed in a plurality at an equal distance from one another in the perimeter of the protruding section.

### [Item 3]

The injection needle of item 1 or 2, wherein the protruding section is disposed substantially coaxial with the hub body, a size of an outer diameter of the protruding section is smaller than a size of an outer diameter of the hub body, and a stepped surface is formed on the tip surface of the hub body.

### [Item 4]

The injection needle of any one of items 1 to 3, wherein the protruding section has a flat tip surface.

### [Item 5]

The injection needle of any one of items 1 to 4, wherein the tip of the needle is cut diagonally with respect to a longitudinal direction of the needle to form an outlet for a drug solution at the tip of the needle, and the outlet of each needle is oriented to substantially face outward in a radial direction.

### [Item 6]

The injection needle of any one of items 1 to 4, wherein the tip of the needle is cut diagonally with respect to a longitudinal direction of the needle to form an outlet for a drug solution at the tip of the needle, and the outlet of each needle is oriented to substantially face inward in a radial direction.

### [Item 7]

An injection needle including a hub and a plurality of needles protruding out from a tip surface of the hub, wherein:
the hub has a hub body formed in a generally tubular shape and a protruding section formed on a tip section of the hub body,
a plurality of longitudinal grooves extending in an axial direction are formed in the perimeter of the protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside of the recessed section is filled, and
a tip of the needle protrudes out forward more than a surface of the protruding section, and a base section of the needle faces a drug solution accommodating section formed inside the hub body.

### [Item 8]

An injection needle to be attached to a tip section of a syringe including a hub and a plurality of needles protruding out from a tip surface of the hub, wherein:
the hub has a hub body formed in a generally tubular shape, an annular protruding section formed on a tip section of the hub body, and a flange formed at a base section of the hub body,
a plurality of longitudinal grooves extending in an axial direction are formed on an inner circumferential surface of the annular protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside the recessed section is filled, and
a tip of the needle protrudes out forward more than a surface of the protruding section, and a base section of the needle faces a drug solution accommodating section formed inside the hub body.

### [Item 9]

An injection needle to be attached to a tip section of a syringe including a hub and a plurality of needles protruding out from a tip surface of the hub, wherein:
the hub has a hub body formed in a generally tubular shape, a height restricting section formed on a tip section of the hub body, and a flange formed at a base section of the hub body,
the height restricting section has a plurality of restriction fragments disposed between each of the plurality of needles,
a tip surface of the restriction fragment is formed to be generally flat, and
a tip of the needle protrudes out forward more than a surface of the height restricting section, and a base section of the needle faces a drug solution accommodating section formed inside the hub body.

### [Item 10]

The injection needle of item 9, wherein there are three needles, and a cross-sectional shape of the height restricting section has a three-point star shape.

### [Advantageous Effects of Invention]

According to the present invention, due to a protruding section protruding out from a tip surface of a hub body, where a needle is secured to the hub body by a fixation agent and the tip of the needle protruding out forward more than the surface of the protruding section, the length of protrusion of the needle from a surface of the protruding section is not affected by the amount of fixation agent applied for fixing the needle to the hub. For this reason, the length of protrusion of the needle is always constant, even if there is variation in the amount of fixation agent that is applied. Therefore, a drug solution can be administered to a location at a depth of interest in an intradermal tissue.

In this manner, an integrated injection needle with a constant length of needle protrusion can be provided, regardless of the bulge in the adhesive.

Further, the needle is disposed in a longitudinal groove formed in the perimeter of the protruding section so that the needle is supported by the protruding section and the position of the needle is stabilized. In particular, when a thin needle is used to alleviate pain, the needle would be more readily bent upon use. However, since the tip section of the needle is supported by being in the longitudinal section, it is possible to prevent the needle from bending and breaking.

Further, since the surface area of the protruding section is relatively large, the area of contact with skin is large. Thus, pain would not be felt when pressing the protruding section against the skin.

In this manner, according to the present invention, skin can be punctured while alleviating pain by making a needle thin. In addition, the length of protrusion of the needle from the surface of the hub body can be made constant. Further, it is possible to prevent deformation of the needle.

Furthermore, a height restricting section is formed at the tip section of the hub body, and the height restricting section has a plurality of restriction fragments disposed between each of a plurality of needles. In addition, the tip surface of the restriction fragment is formed to be generally flat, and the tip of the needle protrudes out forward more than the surface of the height restricting section. Due to the above configuration, the length of protrusion of the needle from the surface of the height restricting section is not affected by the amount of fixation agent applied for fixing the needle to the hub, and therefore the length of protrusion of the needle is always constant, even if there is variation in the amount of fixation agent applied. Therefore, a drug solution can be administered to a location at a depth of interest in an intradermal tissue. Moreover, since a large area of the height restricting section contacts the skin, there is no risk of damaging the skin.

### [Brief Description of Drawings]

[Figure 1] Figure **1** is a cross-sectional view of an injection needle according to one Example of the present invention.
[Figure 2] Figure **2** is a perspective view of main sections of the injection needle shown in Figure **1****.**
[Figure 3] Figures **3** is a cross-sectional view of main sections of the injection needle shown in Figure **1****.**
[Figure 4] Figure **4** is a diagram showing a state of use of the injection needle shown in Figure **1****.**
[Figure 5] Figures **5** is a cross-sectional view of main sections of an injection needle in another Example of the present invention.
[Figure 6] Figure **6** is a perspective view of an injection needle in yet another Example of the present invention.
[Figure 7] Figure **7** is a front view of the injection needle shown in Figure **6****.**
[Figure 8] Figure **8** is a side view of the injection needle shown in Figure **6****.**
[Figure 9] Figure **9** is a cross-sectional view of the injection needle shown in Figure **6****.**
[Figure 10] Figure **10** is a top view of the injection needle shown in Figure **6****.**
[Figure 11] Figure **11** is an enlarged cross-sectional view of main sections of the injection needle shown in Figure **6****.**

### [Description of Embodiments]

The Embodiments of the present invention are hereinafter explained in detail while referring to the drawings.

### (Example 1)

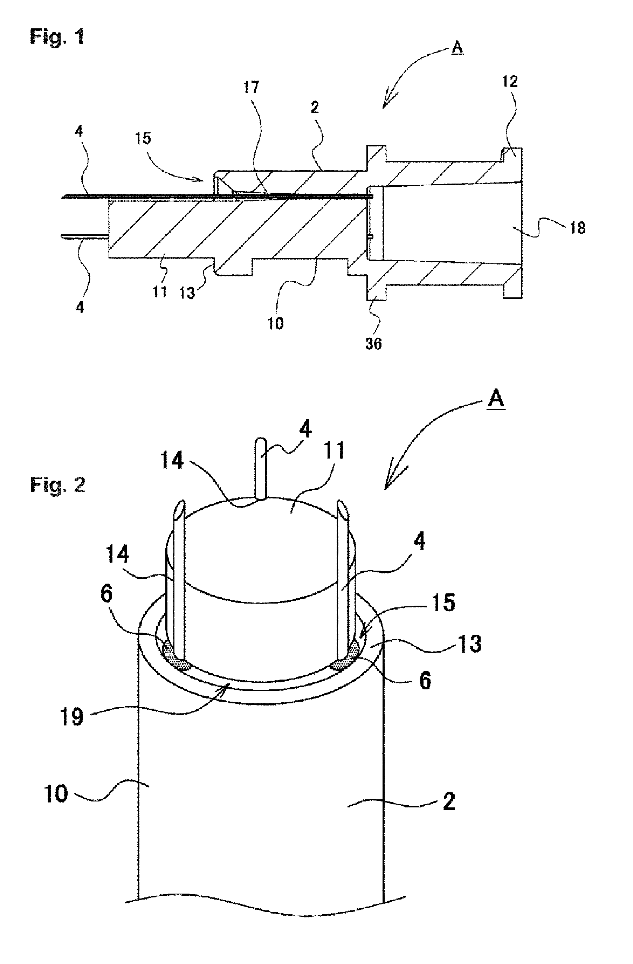

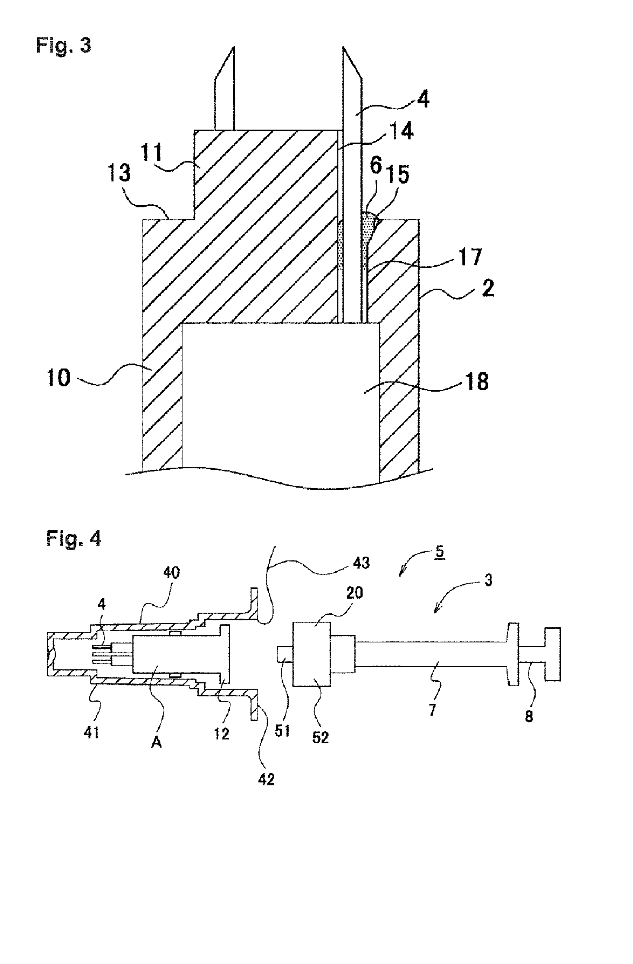

As shown in Figure **4****,** an injection needle **A** of the present invention is attached to the tip section of a syringe **3.** As shown in Figures **1-3** **,** the injection needle **A** has a hub **2** and a plurality of needles **4** protruding out from the tip surface of the hub **2.**

The hub **2** has a hub body **10** formed in a generally tubular shape with an opening at the base end side, a protruding section 11 formed at the tip section of the hub body **10,** and a flange **12** formed at the base section of the hub body **10.** The flange **12** can engage with a Luer lock section **20** of the syringe **3.**

The hub body **10** is formed in a generally cylindrical shape. Further, the protruding section **11** is formed in a generally columnar shape. The protruding section **11** is disposed in a position that is coaxial with the hub body **10.** The size of the outer diameter of the protruding section **11** is formed smaller than the size of the outer diameter of the hub body **10,** resulting in a stepped surface **13** being formed around the tip of the hub body **10.**

The tip surface of the protruding section **11** is formed to be generally flat. The tip surface of the protruding section **11** may be formed to be slightly protruding and curved.

A plurality of longitudinal grooves **14** extending in the axial direction are formed in the perimeter of the protruding section **11.** The needle **4** is disposed within the longitudinal groove **14.** The number of longitudinal grooves **14** can be changed in accordance with the number of needles **4.** The cross-sectional shape of the longitudinal grooves **14** preferably has a generally semi-circular shape in order to support the needles **4.** Further, the longitudinal grooves **14** can be formed in a plurality at equal distance from one another in the perimeter of the protruding section **11.**

A through hole **17** for allowing the needle **4** to pass through is formed in the hub body **10** at a position where the above-described longitudinal groove **14** is extended. The through hole **17** is formed along the axial direction of the hub body **10.** The inner diameter of the through hole **17** is formed to be slightly larger than the outer diameter of the needle **4.**

A recessed section **15** is formed in the stepped surface **13** of the hub body **10** in the perimeter of the through hole **17,** through which the needle **4** passes. The inside of the recessed section 15 is filled with a fixation agent **6,** such as an adhesive. The needle **4** is secured to the hub body **10** by the fixation agent **6.**

An annular recessed groove **19** is formed on the stepped surface 13 in the perimeter of the base section of the protruding section **11.** The annular recessed groove **19** can be continuous with the recessed section **15.** The annular recessed groove **19** is formed across the entire circumference of the stepped surface **13.** Although the cross-sectional shape of the recessed section **15** can be generally triangular as shown in Figure **3****,** the cross-sectional shape may have any shape, such as a trough-like shape.

In this manner, the base section of the needle **4** disposed in the longitudinal groove **14** of the protruding section **11** passes through the through hole **17,** and the needle **4** is fixed to the hub body **10** by the fixation agent **6** such as an adhesive, with which inside of the recessed section **15** is filled. The injection needle is configured so that the fixation agent **6** can be poured into and applied within the through hole **17** through the recessed section **15.**

Even when there is an excessive amount of the fixation agent 6 filled within the recessed section **15** such that the solidportion thereof bulges out, the solid portion of the fixation agent **6** never exceeds the protruding section **11.** Further, the excessive amount of the fixation agent **6** can also flow into the annular recessed groove **19** from the recessed section **15.**

The fixation agent **6** may also be applied to the longitudinal groove **14** to have the needle **4** to be fixed to the outer circumferential surface of the protruding section **11.** Further, fixation methods of the needle **4** are not limited to adhesion. For example, a method such as fusion (thermal fusion, ultrasonic fusion, or high-frequency fusion) may be used.

As shown in Figure **2****,** the tip of the needle **4** protrudes out forward more than the surface of the protruding section **11.** The length of the needle **4** protruding out from the tip surface of the protruding section **11** is preferably 3 mm or less, more preferably about 0.5 to 2.0 mm, and most preferably about 0.5 to 1.5 mm.

The maximum outer diameter of the needle **4** is preferably about 0.1 to 0.6 mm and more preferably about 0.1 to 0.3 mm.

Further, the number of needles **4** is preferably 2 to 10.

The base portion of the needle **4** faces a drug solution accommodating section **18** formed inside the hub body 10. Thus, the inside of the needle **4** communicates with the inside of the drug solution accommodating section **18.** The base section side of the hub body **10** of the drug solution accommodating section **18** is open.

The tip of the needle **4** described above is cut diagonally with respect to the longitudinal direction of the needle **4** to form an outlet for a drug solution at the tip of the needle **4.** In addition, an outlet of each of the plurality of needles **4** provided in the longitudinal groove **14** that is formed in the perimeter of the protruding section **11** is oriented to generally face outward in the radial direction.

The material constituting the needle **4** includes, but not limited to, metallic materials such as stainless steel, aluminum or aluminum alloy, and titanium or titanium alloy.

The hub **2** can be manufactured by injection molding using a thermoplastic resin such as polycarbonate, polypropylene, ABS resin, or polystyrene as the material.

As shown in Figure **4****,** a percutaneous drug administering device 5 is formed by attaching the injection needle **A** of the present invention to the tip section of the syringe **3.**

The syringe **3** has a syringe body **7,** a plunger **8** inserted into the syringe body **7,** and a Luer lock section **20** formed at the tip section of the syringe body **7.** The Luer lock section **20** has an inner tube **51** and an outer tube **52.** The inner surface of the outer tube **52** is threaded.

A cap **40** for housing the injection needle **A** has a cap body **41** formed in generally a cylindrical shape with one end open, and a flange **42** formed on the open end side of the cap body **41.** Four grooves are provided in the axial direction on the inner surface of the cap body.

The injection needle **A** is housed within the cap **40** while a protrusion **36** formed on the outer surface of the injection needle **A** engages with a groove of the cap **40.** The injection needle **A** is housed within the cap **40** in a sterile state by a sealing member **43** affixed to the flange **42.**

The following descriptions explain a method of operating the percutaneous drug administering device **5.**

When the injection needle **A** is used, the sealing member **43** affixed to the flange **42** of the cap **40** is peeled off. The Luer lock section **20** at the tip of the syringe **3** is engaged with the flange **12** at the rear end section of the injection needle **A** and the syringe **3** is rotated. Since the injection needle **A** cannot be rotated with respect to the cap **40,** the Luer lock section **20** of the syringe **3** engages with the injection needle **A.** The injection needle **A** is pulled out from the cap **40** and used in this state.

In accordance with conventional methods, the plunger **8** of the syringe **3** is then operated to be pressed in, whereby a drug solution within the syringe **3** enters the drug solution accommodating section **18** of the injection needle **A** and is pushed out from the drug solution outlet through the needle **4.**

When the needle tip is pressed against the skin at this time, the needle **4** enters the skin. Since the tip surface of the protruding section **11** contacts the skin, the needle stops in this state. Thus, the needle **4** can enter the skin only by a predetermined length.

Each of the needles **4** is disposed within a longitudinal groove **14** formed on the outer circumferential surface of the protruding section **11.** Thus, each of the needles **4** is supported by the protruding section **11,** by which deformation of the needles is prevented.

Further, each of the needles **4** tends to move towards the center in the radial direction by the effect of a drug solution being pushed out into the skin. However, since the movement of the needle **4** is prevented by the protruding section **11,** movement of the needle **4** within the skin is prevented. Thus , a drug solution can be supplied intradermally to an extensive region. In other words, a drug solution is not delivered to deep parts of the skin.

### (Example 2)

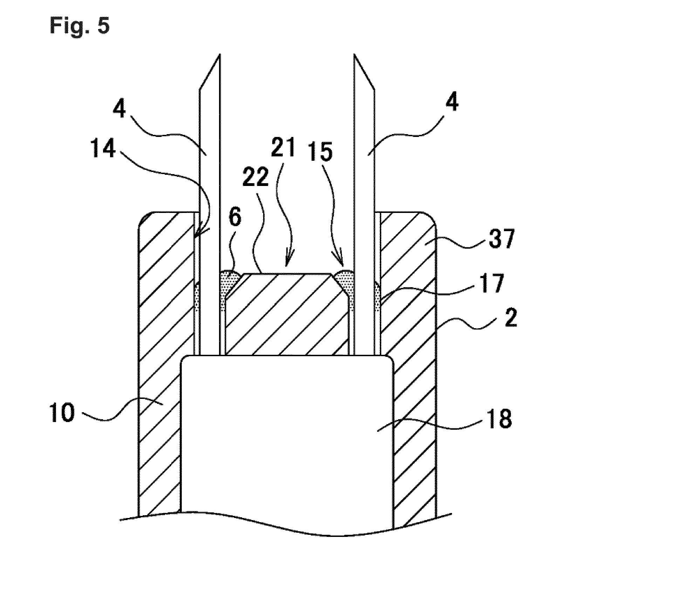

As shown in Figure **5****,** a hub **2** has a hub body **10** formed in a generally tubular shape with an opening at the base end side, a protruding section **37** formed at the tip section of the hub body **10,** and a flange formed at the base section of the hub body **10.** The flange can engage with a Luer lock section **20** of a syringe **3.**

The protruding section **37** is formed in an annular shape. In addition, a cylindrical recess **21** is formed on the inside of the annular protruding section **37.** The protruding section **37** is disposed at a position that is concentric with the hub body **10.** A center tip surface **22** of the hub body **10** is formed on the bottom surface of the recess **21.**

The tip surface of the protruding section 37 is formed to be generally flat.

A plurality of longitudinal grooves **14** extending in the axial direction are formed on the inner circumferential surface of the protruding section **37.** The needle **4** is disposed within the longitudinal groove **14.** The number of longitudinal grooves **14** can be changed in accordance with the number of needles **4.**

The cross-sectional shape of the longitudinal groove **14** is preferably semi-circular. Further, multiple longitudinal grooves **14** can be formed at equal distance from one another on the inner circumferential surface of the protruding section **37.**

A through hole **17** for allowing a needle to pass through the hub body 10 is formed at a position for extending the longitudinal groove **14.** The through hole **17** is formed along the axial direction of the hub body **10.**

The needle **4** is passed through the through hole **17.** The inner diameter of the through hole **17** is formed to be slightly larger than the outer diameter of the needle **4.**

A recessed section **15** is formed on the center tip section **22** of the hub body **10** within the recess **21** in the perimeter of the through hole **17**, through which the needle **4** passes . The inside of the recessed section **15** is filled with a fixation agent **6** such as an adhesive, by which the needle **4** is fixed to the hub body **10.**

An annular recessed groove may be formed in the perimeter of the central tip surface **22** of the hub body **10** described above. The annular recessed groove is continuous with the recessed section **15.**

In this manner, the needle **4** disposed within the longitudinal groove **14** of the annular protruding section **37** passes through the through hole **17,** and the needle **4** is fixed to the hub body **10** by the fixation **agent 6** such as an adhesive with which the recessed section **15** is filled.

The injection needle is configured so that the fixation agent **6** can be applied within the through hole **17** through the recessed section **15.** Further, the injection needle may be configured so that multiple needles are fixed with a single application of the fixation agent **6** by applying the fixation agent **6** to the annular recessed groove within the recess **21.**

The length of protrusion of the needle **4** from the surface of the annular protruding section **37** is also restricted in the present Example. The length of protrusion of the needle **4** is not affected by the amount of application of adhesive or the like for securing the needle **4.** Further, each of the needles **4** is disposed in the longitudinal groove **14** formed on the inner surface of the annular protruding section **37.** Thus, deformation or slanting of the needle **4** can be inhibited upon use.

In each of the above-described Examples, an outlet of a needle is configured to be oriented outward in the radial direction. However, the outlet may be oriented inward in the radial direction. Since a drug solution pushed out from an outlet of a needle is pushed out towards the center direction of the hub, an outward force in the radial direction is applied to the tip section of each needle. However, deformation of the needle can be prevented by supporting the needle with the annular protruding section **37.**

### (Example 3)

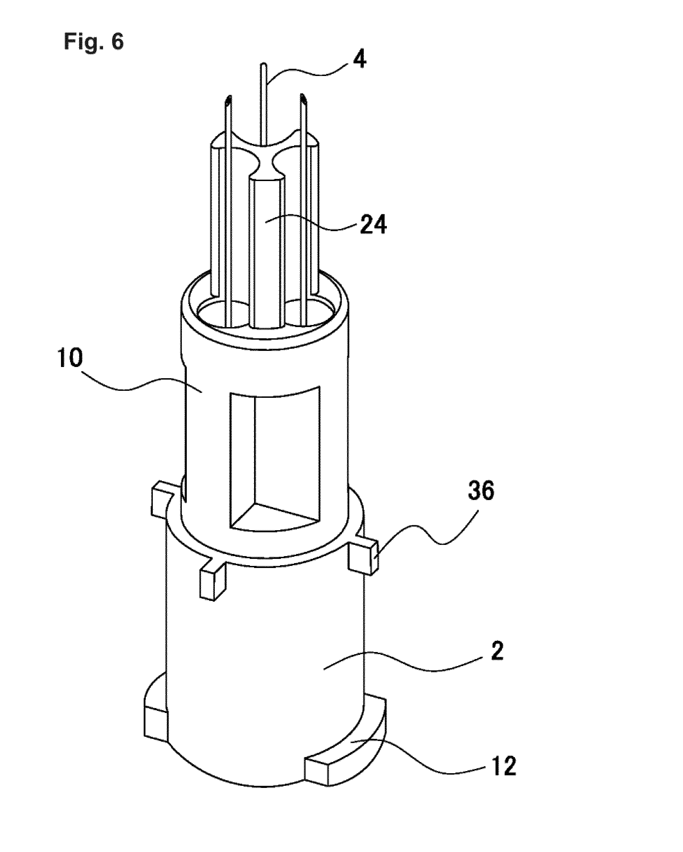

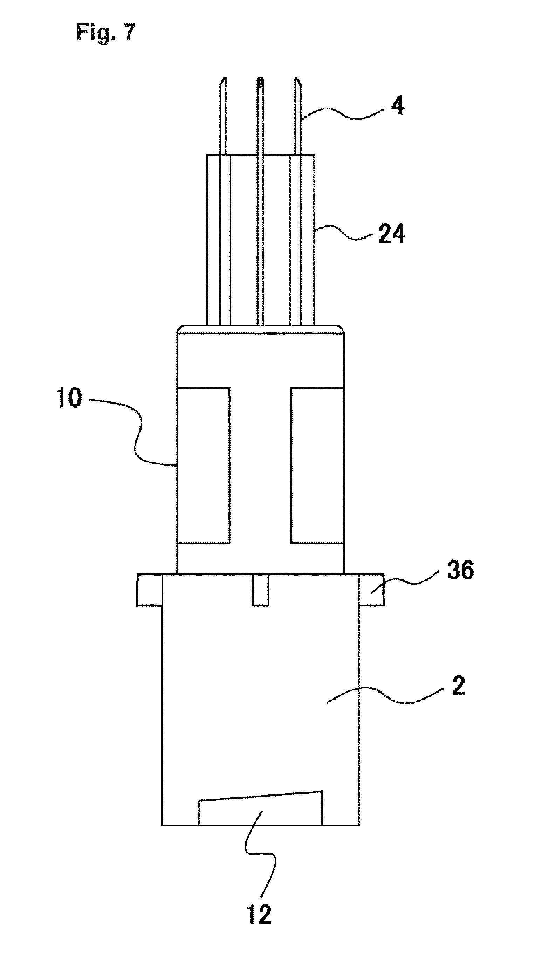

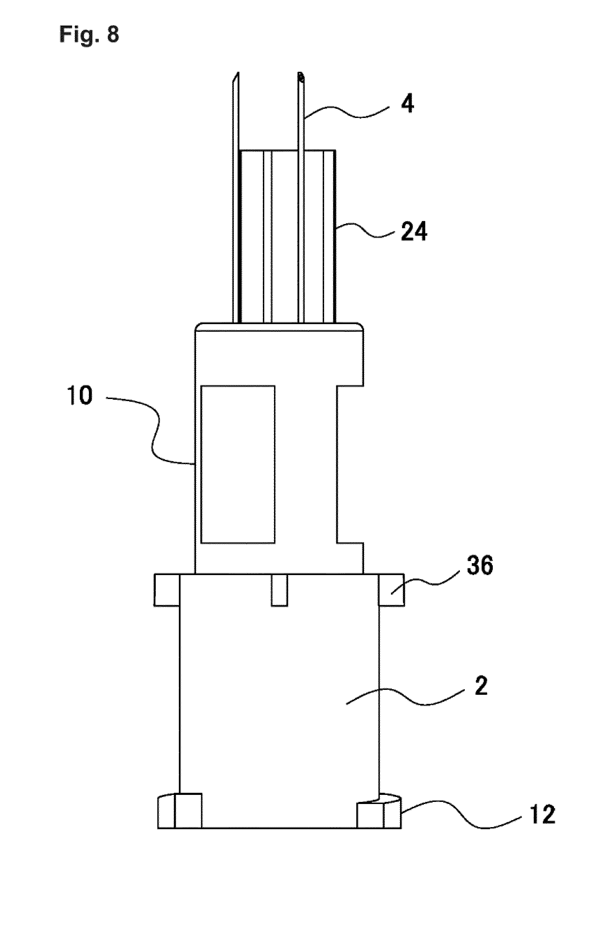

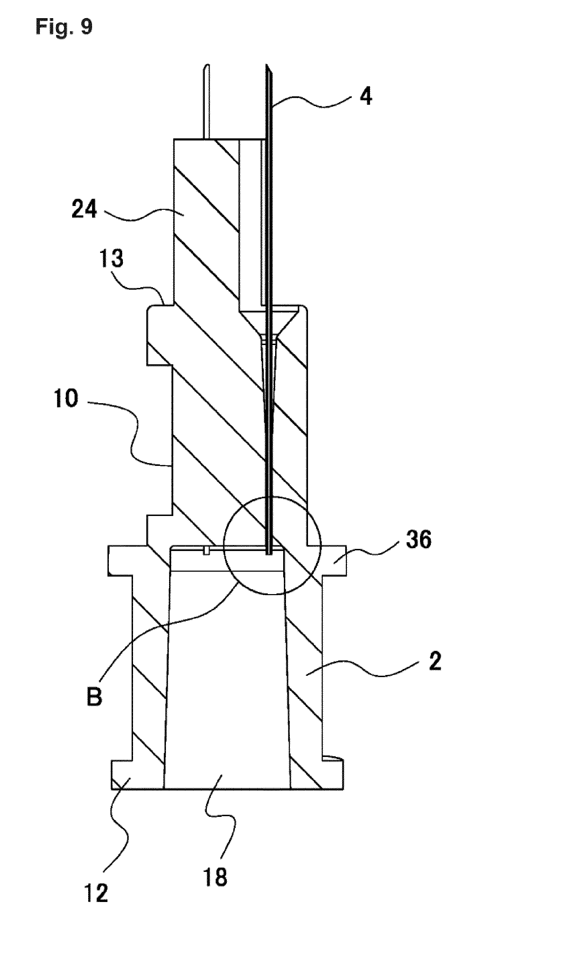

In this Example, as shown in Figures **6-11****,** a hub **2** has a hub body **10,** a height restricting section **24** protruding out from the tip section of the hub body **10,** a plurality of needles **4** that protrude out from the tip section of the hub body **10,** and a flange **12** formed at the base section of the hub body **10.** The height restricting section **24** is provided between the plurality of needles **4.**

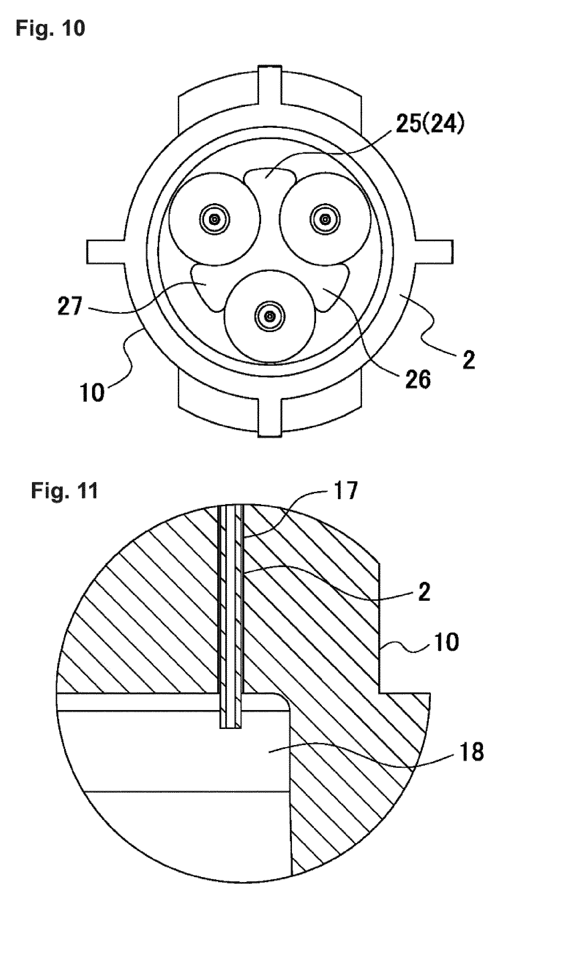

The cross-section of the restricting section **24** is generally a three-point star shape. The height restricting section **24** consists of a first restriction fragment **25,** a second restriction fragment **26,** and a third restriction fragment **27,** whose center sections are connected. Each of the restriction fragments **25-27** is disposed so that each of the restriction fragments **25-27** is separated by 120 degrees in plan view (Figure **10**).

The first to third restriction fragments **25-27** are disposed between each of the needles **4.** The tip surface of the restricting section **24** is formed to be generally flat.

The length of protrusion of the needle **4** from the tip surface of the restricting section **24,** the size and number of the needle **4,** and the like can be designed similarly to the above-described Example 1.

In this Example, it is also possible to integrally form the restricting section **24** with the hub body **10.**

Three needles **4** were formed equidistantly from the center and at locations separated by 120 degrees on the tip surface of the injection needle **A.** However, the arrangement and number of needles **4** are not limited thereto.

### [Industrial Applicability]

The present invention provides an injection needle capable of supplying a drug solution to a tissue layer at a depth of interest. Further, an integrated injection needle with a constant length of needle protrusion, regardless of the bulge of an adhesive, can be provided.

### [Reference Signs List]

- **A**: injection needle
- **2**: hub
- **3**: syringe
- **4**: needle
- **5**: percutaneous drug administering device
- **6**: fixation agent
- **10**: hub body
- **11**: protruding section
- **12**: flange
- **14**: longitudinal groove
- **15**: recessed section
- **17**: through hole
- **18**: drug solution accommodating section
- **40**: cap

## Claims

1. An injection needle to be attached to a tip section of a syringe, including a hub and a plurality of needles protruding out from a tip surface of the hub, wherein:
the hub has a hub body formed in a generally tubular shape, a protruding section formed on a tip section of the hub body, and a flange formed at a base section of the hub body,
a plurality of longitudinal grooves extending in an axial direction are formed in the perimeter of the protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside of the recessed section is filled, and
a tip of the needle protrudes out forward more than a surface of the protruding section, and a base section of the needle faces a drug solution accommodating section formed inside the hub body.

2. The injection needle of claim 1, wherein the longitudinal grooves are formed in a plurality at an equal distance from one another in the perimeter of the protruding section.

3. The injection needle of claim 1, wherein the protruding section is disposed substantially coaxial with the hub body, a size of an outer diameter of the protruding section is smaller than a size of an outer diameter of the hub body, and a stepped surface is formed on the tip surface of the hub body.

4. The injection needle of claim 1, wherein the protruding section has a flat tip surface.

5. The injection needle of claim 1, wherein the tip of the needle is cut diagonally with respect to a longitudinal direction of the needle to form an outlet for a drug solution at the tip of the needle, and the outlet of each needle is oriented to substantially face outward in a radial direction.

6. The injection needle of claim 1, wherein the tip of the needle is cut diagonally with respect to a longitudinal direction of the needle to form an outlet for a drug solution at the tip of the needle, and the outlet of each needle is oriented to substantially face inward in a radial direction.

7. An injection needle including a hub and a plurality of needles protruding out from a tip surface of the hub, wherein:
the hub has a hub body formed in a generally tubular shape and a protruding section formed on a tip section of the hub body,
a plurality of longitudinal grooves extending in an axial direction are formed in the perimeter of the protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside of the recessed section is filled, and
a tip of the needle protrudes out forward more than a surface of the protruding section, and a base section of the needle faces a drug solution accommodating section formed inside the hub body.

8. An injection needle to be attached to a tip section of a syringe including a hub and a plurality of needles protruding out from a tip surface of the hub, wherein:
the hub has a hub body formed in a generally tubular shape, an annular protruding section formed on a tip section of the hub body, and a flange formed at a base section of the hub body,
a plurality of longitudinal grooves extending in an axial direction are formed on an inner circumferential surface of the annular protruding section,
a through hole is formed on the hub body so as to extend the longitudinal groove, the needle disposed in the longitudinal groove of the protruding section being passed through the through hole,
a recessed section is formed on a tip surface of the hub body in the perimeter of the through hole through which the needle is passed, the needle being fixed with a fixation agent with which inside of the recessed section is filled, and
a tip of the needle protrudes out forward more than a surface of the protruding section, and a base section of the needle faces a drug solution accommodating section formed inside the hub body.

9. An injection needle to be attached to a tip section of a syringe including a hub and a plurality of needles protruding out from a tip surface of the hub, wherein:
the hub has a hub body formed in a generally tubular shape, a height restricting section formed on a tip section of the hub body, and a flange formed at a base section of the hub body,
the height restricting section has a plurality of restriction fragments disposed between each of the plurality of needles,
a tip surface of the restriction fragment is formed to be generally flat, and
a tip of the needle protrudes out forward more than a surface of the height restricting section, and a base section of the needle faces a drug solution accommodating section formed inside the hub body.

10. The injection needle of claim 9, wherein there are three needles, and a cross-sectional shape of the height restricting section has a three-point star shape.
